(19) Europäisches Patentamt
European Patent Office
Office européen des brevets

(11) EP 1 865 918 B1

(12) **FASCICULE DE BREVET EUROPEEN**

(45) Date de publication et mention de la délivrance du brevet:
**06.05.2009 Bulletin 2009/19**

(21) Numéro de dépôt: **06725138.9**

(22) Date de dépôt: **17.03.2006**

(51) Int Cl.:
***A61K 8/81*** *(2006.01)* ***A61Q 19/08*** *(2006.01)*
***A61Q 19/00*** *(2006.01)* ***A61Q 19/10*** *(2006.01)*

(86) Numéro de dépôt international:
**PCT/EP2006/060843**

(87) Numéro de publication internationale:
**WO 2006/097529 (21.09.2006 Gazette 2006/38)**

(54) **UTILISATION COSMETIQUE D'UN COPOLYMERE PARTICULIER EN TANT QU'AGENT TENSEUR DE LA PEAU DANS UNE COMPOSITION COSMETIQUE**

KOSMETISCHE VERWENDUNG EINES BESTIMMTEN COPOLYMERS ALS HAUTSPANNER IN EINER KOSMETISCHEN ZUSAMMENSETZUNG

COSMETIC USE OF A PARTICULAR COPOLYMER AS SKIN TENSOR IN A COSMETIC COMPOSITION

(84) Etats contractants désignés:
**DE ES FR GB IT**

(30) Priorité: **17.03.2005 FR 0550692**

(43) Date de publication de la demande:
**19.12.2007 Bulletin 2007/51**

(73) Titulaire: **L'Oréal**
**75008 Paris (FR)**

(72) Inventeur: **CASSIN, Guillaume**
**F-91140 Villebon Sur Yvette (FR)**

(74) Mandataire: **Ilgart, Jean-Christophe**
**BREVALEX**
**3, rue du Docteur Lancereaux**
**75008 Paris (FR)**

(56) Documents cités:
**WO-A-02/15875**
**FR-A- 2 848 557**
**FR-A- 2 838 345**
**US-A- 6 113 882**

EP 1 865 918 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### DOMAINE TECHNIQUE

La présente invention a trait à l'utilisation cosmétique d'un copolymère comprenant des unités dérivant du styrène et des unités dérivant du (méth)acrylate d'éthyle en tant qu'agent tenseur de la peau dans une composition cosmétique.

La présente invention a également trait à un procédé cosmétique pour atténuer les rides d'une peau ridée consistant à appliquer sur ladite peau une composition comprenant, dans un milieu physiologiquement acceptable, au moins un copolymère comprenant des unités dérivant du styrène et des unités dérivant du (méth)acrylate d'éthyle tel que mentionné ci-dessus.

Le domaine général de l'invention est donc celui du vieillissement de la peau.

Au cours du vieillissement de la peau, il apparaît différents signes se traduisant notamment par une modification de la structure et des fonctions cutanées. L'un de ces principaux signes est l'apparition de ridules et de rides profondes dont l'importance et le nombre croissent avec l'âge. Le microrelief de la peau devient moins régulier et présente un caractère anisotrope.

### ETAT DE LA TECHNIQUE ANTERIEURE

Il est courant de traiter ces signes de vieillissement par des compositions cosmétiques contenant des actifs susceptibles de lutter contre le vieillissement, tels que des $\alpha$-hydroxyacides, des $\beta$-hydroxyacides et des rétinoïdes. Ces actifs agissent notamment sur les rides en éliminant les cellules mortes de la peau et en accélérant le processus de renouvellement cellulaire. Cependant, ces actifs présentent l'inconvénient de n'être efficaces pour le traitement des rides qu'après un certain temps d'application, à savoir un temps pouvant aller de quelques jours à plusieurs semaines.

Or, les besoins actuels tendent de plus en plus vers l'obtention de compositions permettant d'obtenir un effet immédiat, conduisant rapidement à un lissage des rides et/ou ridules et à la disparition, même temporaire, des marques de fatigue. De telles compositions sont des compositions comprenant des agents tenseurs.

Ces agents tenseurs peuvent être notamment des polymères d'origine naturelle ou synthétique en dispersion aqueuse, capables de former un film provoquant la rétraction du *stratum corneum,* qui correspond à la couche cornée superficielle de l'épiderme.

Ainsi, en tant qu'agents tenseurs susceptibles de présenter un effet immédiat, les documents WO 98/29092 [1] et EP 1 038 519 [2] décrivent des dispersions aqueuses d'un système polymérique comprenant au moins un polymère synthétique. Les polymères synthétiques mentionnés se présentent sous la forme de latex ou d'un pseuso-latex, par exemple de type polycondensat tel que des polyuréthannes anioniques, cationiques, non ioniques ou amphotères, les polyuréthannes-acryliques, les polyuréthannes-polyvinylpyrrolidones, les polyesters-polyuréthannes, les polyéthers-polyuréthannes, les polyurées et leurs mélanges. Il est également décrit des latex de polymère siliconé greffé comprenant une portion siloxane et une portion constituée d'une chaîne organique non siliconée, l'une des deux portions constituant la chaîne principale du polymère et l'autre étant greffée sur ladite chaîne principale. Un tel polymère siliconé greffé (encore désigné par polysilicone 8-selon la nomenclature CTFA) est notamment commercialisé par la Société 3M sous la dénomination commerciale VS 80.

Le document FR 2 828 810 [3] décrit des compositions à effet tenseur comprenant en tant qu'agent tenseur un polymère d'origine naturelle constitué par un polysaccharide éventuellement sous forme d'un extrait végétal, en particulier un extrait d'algues et un agent hydratant polyhydroxylé.

Le document FR 2822676 [4] décrit des compositions cosmétiques filmogènes lissant la peau et comprenant au moins à titre d'agent filmogène un copolymère acrylique, dont les unités monomères sont choisies parmi l'acide acrylique, l'acide méthacrylique, les (méth)acrylates d'alkyle comprenant de préférence jusqu'à 30 atomes de carbone.

Les compositions antirides de l'art antérieur, notamment celles décrites ci-dessus, ont pour particularité d'entraîner souvent l'apparition d'un film laqué inesthétique, après application de ces compositions sur la peau.

Il existe donc un besoin pour un agent tenseur qui, lorsqu'il est incorporé dans une composition cosmétique, fasse en sorte que la composition résultante présente des propriétés de lissage mécanique de la peau efficaces et empêche l'apparition d'un film laqué sur la peau.

De manière surprenante, les Inventeurs ont découvert qu'en incorporant un copolymère particulier dans des compositions antirides, les compositions résultantes présentent les propriétés attendues définies dans le paragraphe ci-dessus.

### EXPOSÉ DE L'INVENTION

Ainsi, l'invention a trait, selon un premier objet, à l'utilisation cosmétique en tant qu'agent tenseur de la peau

dans une composition cosmétique d'un copolymère comprenant des unités dérivant du styrène et des unités dérivant du (méth)acrylate d'éthyle, dans lequel le rapport en poids entre les unités dérivant du styrène et les unités dérivant du (méth)acrylate d'éthyle est supérieur ou égal à 1.

Selon l'invention, on précise que par unité dérivant du styrène, on entend une unité obtenue directement à partir du monomère styrène par polymérisation, c'est-à-dire une unité de formule suivants :

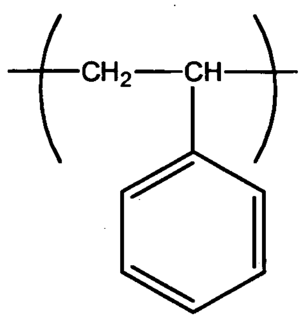

Selon l'invention, on précise que par unité dérivant du (méth)acrylate d'éthyle, on entend une unité obtenue directement à partir du monomère acrylate d'éthyle, auquel cas l'unité répond à la formule suivants :

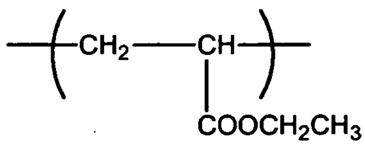

ou une unité obtenue directement à partir du monomère méthacrylate d'éthyle, auquel cas l'unité répond à la formule suivants :

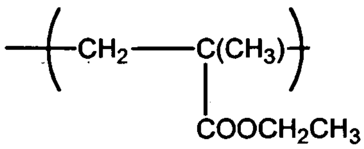

On précise que par agent tenseur, on entend, dans ce qui précède et ce qui suit, un composé susceptible d'avoir un effet tenseur, c'est-à-dire un composé pouvant tendre la peau et par cet effet de tension lisser la peau et faire diminuer voire disparaître de façon immédiate les rides et les ridules.

En plus de présenter un effet tenseur très efficace, les copolymères de l'invention ont pour particularité de ne pas engendrer d'effets inesthétiques, tels que l'apparition d'un film laqué brillant. Ils permettent également de diminuer la visibilité du micro-relief de la peau, procurant ainsi à la peau un effet « soft-focus ».

Outre les unités dérivant du styrène et du (méth)acrylate d'éthyle, les copolymères tels que définis ci-dessus peuvent comprendre des unités dérivant de monomères choisis parmi l'acide (méth)acrylique, le (méth) acrylate de méthyle, le (méth)acrylate de butyle, le (méth)acrylate d'éthylhexyle, le (méth)acrylate de 2-hydroxyéthyle.

Les copolymères utilisés selon l'invention peuvent être des copolymères aléatoires, c'est-à-dire des copolymères comportant une répartition aléatoire des unités dérivant du styrène et dérivant du (méth)acrylate d'éthyle, et éventuellement des autres unités. Plus précisément, cela signifie que la probabilité de trouver une unité monomère donnée (styrène, (méth)acrylate d'éthyle et éventuellement d'autres unités) en un site donné quelconque de la chaîne est indépendante des unités adjacentes.

Les copolymères utilisés selon l'invention peuvent être des copolymères statistiques.

Enfin, les copolymères utilisés selon l'invention peuvent être également selon l'invention des copolymères à blocs, ce qui constitue l'alternative préférentielle de l'invention.

Avantageusement, les copolymères à blocs utilisés selon l'invention sont des copolymères linéaires à blocs du type A-[B-A]$_n$ ou B-[A-B]$_n$ ou [A-B]$_n$ dans lesquels A est un bloc comprenant au moins 50% en poids d'unités dérivant du styrène, B est un bloc comprenant au moins 50% en poids d'unités dérivant du (méth)acrylate d'éthyle, et n est un nombre supérieur ou égal à 1.

Les blocs A et B s'enchaînent linéairement. Si n=1 le copolymère est un copolymère tribloc A-B-A ou B-A-B, ou un copolymère dibloc A-B. Si n=2, le copolymère est un copolymère A-B-A-B-A, ou B-A-B-A-B ou A-B-A-B. Si n≥3, il ne s'agit pas d'un copolymère étoile ou téléchélique dans lequel un bloc A ou un bloc B constituerait un coeur. De préférence, n =1, plus préférablement le copolymère est un copolymère tribloc A-B-A. On note que le copolymère peut comprendre des fonctions ou groupes de polymérisation ou des résidus de tels fonctions ou groupes, en bout de chaînes macromoléculaires. Il peut s'agir par exemple de groupes de transferts, ou de résidus de groupes de transferts, comprenant par exemple un groupe de formule -S-CS-, ou un résidu de ce groupe.

Selon le mode de réalisation particulier mentionné ci-dessus, le bloc A comprend au moins 50% en poids d'unités dérivant du styrène, par rapport au poids total du bloc A. Le bloc A peut comprendre d'autres unités que celles dérivant du styrène, pouvant être destinées à moduler des propriétés du copolymère ou à faciliter sa préparation. Le bloc A peut donc être un copolymère statistique comprenant des unités dérivant de styrène et d'autres unités. On peut ainsi moduler la solubilité du bloc A dans l'eau ou dans d'autres milieux, ou moduler sa température de transition vitreuse et ainsi moduler sa rigidité. Les autres unités du bloc A peuvent être des unités dérivant de monomères choisis parmi l'acide acrylique, l'acide méthacrylique, l'acrylate de méthyle, de butyle, d'ethylhexyle, ou de 2-hydroxyéthyle, ou de méthacrylate de méthyle, de butyle, d'ethylhexyle, ou de 2-hydroxyéthyle. La présence de faibles quantités d'acide méthacrylique peut notamment faciliter la préparation du copolymère, le bloc A peut par exemple en comprendre de 0,1 % à moins de 5 % en poids, par rapport au poids total du bloc A. Le bloc A comprend de préférence au moins 75% en poids, de préférence au moins 90% en poids, de préférence au moins 95% en poids, d'unités dérivant de styrène, par rapport au poids total du bloc A.

Dans ce cas, le ou les éventuelles autres unités dérivant de monomères autres que le styrène représentent donc, de préférence, 25% en poids ou moins, de préférence 10% en poids ou moins, de préférence encore 5% en poids ou moins, encore plus préférentiellement environ 2% en poids, par rapport au poids total du bloc A.

Le bloc B tel que défini ci-dessus comprend au moins 50% en poids d'unités dérivant d'acrylate d'éthyle ou de méthacrylate d'éthyle. Le bloc B peut comprendre d'autres unités que celles dérivant de l'acrylate d'éthyle ou du méthacrylate d'éthyle, pouvant être destinées à moduler des propriétés du copolymère ou à faciliter sa préparation. Le bloc B peut donc être un copolymère statistique comprenant des unités dérivant de l'acrylate d'éthyle ou du méthacrylate d'éthyle et d'autres unités. On peut ainsi moduler la solubilité du bloc B dans l'eau ou dans d'autres milieux, ou moduler sa température de transition vitreuse et ainsi moduler sa rigidité. Les autres unités du bloc B peuvent être des unités dérivant de monomères choisis parmi l'acide acrylique, l'acide méthacrylique, l'acrylate de méthyle, de butyle, d'ethylhexyle, ou de 2-hydroxyéthyle, ou de méthacrylate de méthyle, de butyle, d'ethylhexyle, ou de 2-hydroxyéthyle. La présence de faibles quantités d'acide méthacrylique peut notamment faciliter la préparation du copolymère, le bloc B peut par exemple en comprendre de 0,1 % à moins de 5 % en poids, par rapport au poids total du bloc B. Le bloc B peut alternativement ne pas comprendre d'unités dérivant d'acide méthacrylique. Le bloc B comprend de préférence au moins 75% en poids, de préférence au moins 90% en poids, de préférence au moins 95% en poids, d'unités dérivant d'acrylate d'éthyle ou de méthacrylate d'éthyle, par rapport au poids total du bloc B. Dans ce cas, le ou les éventuelles autres unités dérivant de monomères autres que le (méth)acrylate d'éthyle représentent donc, de préférence, 25% en poids ou moins, de préférence 10% en poids ou moins, de préférence encore, 5% en poids ou moins, par rapport au poids total du bloc B.

Le rapport peut être déterminé en faisant le rapport entre les monomères introduits pour la préparation du copolymère, ou en faisant le rapport entre les masses moléculaires moyennes des blocs. De préférence, le rapport entre les masses moléculaires moyennes des blocs est supérieur ou égal à 1, de préférence supérieur ou égal à 1,5, de préférence supérieur ou égal à 2,01, de préférence supérieur ou égal à 2,5, de préférence supérieur ou égal à 5.

On précise que les masses moléculaires moyennes susmentionnées sont des masses moléculaires moyennes théoriques ou « visées » du bloc.

La masse moléculaire moyenne théorique $M_{bloc}$ d'un bloc, est typiquement calculée selon la formule suivante:

$$M_{bloc} = \sum_i M_i * \frac{n_i}{n_{precursor}},$$

où $M_i$ est la masse molaire d'un monomère i, ni est le nombre de moles du monomère i, $n_{precursor}$ est le nombre de moles de fonctions auquel sera liée la chaîne macromoléculaire du bloc. Les fonctions peuvent provenir d'un agent de transfert (ou un groupe de transfert) ou un amorceur, un bloc précédent etc. S'il s'agit d'un bloc précédent, le nombre

de moles peut être considéré comme le nombre de moles d'un composé auquel la chaîne macromoléculaire dudit bloc précédent a été liée, par exemple un agent de transfert (ou un groupe de transfert) ou un amorceur. En pratique, les masses moléculaires moyennes théoriques sont calculées à partir du nombre de moles de monomères introduits et du nombre de moles de précurseur introduit.

La masse moléculaire moyenne mesurée d'un premier bloc ou d'un copolymère désigne la masse moléculaire moyenne en nombre en équivalents polystyrène d'un bloc ou d'un copolymère mesurée par chromatographie d'exclusion stérique (SEC), dans du THF, avec une calibration à l'aide d'étalons de polystyrène. La masse moléculaire moyenne mesurée d'un $n^{ième}$ bloc dans un copolymère à n blocs est définie comme la différence entre la masse moléculaire moyenne mesurée du copolymère et la masse moléculaire moyenne mesurée du copolymère à (n-1) blocs à partir duquel il est préparé.

Selon l'invention, la masse moléculaire moyenne en nombre de chaque bloc, qu'il s'agisse du bloc d'unité styrène ou du bloc d'unité acrylate d'éthyle, est comprise entre 1000 g/mol et 200 000 g/mol, de préférence entre 5000 g/mol et 100 000 g/mol.

Un copolymère à blocs particulièrement avantageux est un copolymère tribloc comprenant:

- un premier bloc comprenant des unités dérivant du styrène ayant une masse moléculaire moyenne en nombre de 30 000 g/mol ;
- un deuxième bloc constitué d'unités dérivant de l'acrylate d'éthyle ayant une masse moléculaire moyenne en nombre de 10 000 g/mol ;
- un troisième bloc comprenant des unités dérivant du styrène ayant une masse moléculaire moyenne en nombre de 30 000 g/mol.

Un copolymère répondant à la définition donnée ci-dessus, peut être un copolymère pour lequel le premier bloc et/ou le troisième bloc et, de préférence, le premier bloc et le troisième bloc comporte, outre les unités dérivant du styrène, des unités dérivant de l'acide méthacrylique, par exemple, dans un rapport massique (styrène/acide méthacrylique) de 98/2.

Un copolymère également particulièrement avantageux est un copolymère tribloc comprenant :

- un premier bloc comprenant des unités dérivant du styrène ayant une masse moléculaire moyenne en nombre de 32 500 g/mol ;
- un deuxième bloc comprenant des unités dérivant de l'acrylate d'éthyle ayant une masse moléculaire moyenne en nombre de 5000 g/mol ;
- un troisième bloc comprenant des unités dérivant du styrène ayant une masse moléculaire moyenne en nombre de 32 500 g/mol.

Un autre copolymère également particulièrement avantageux est un copolymère tribloc comprenant :

- un premier bloc comprenant des unités dérivant du styrène ayant une masse moléculaire moyenne en nombre de 25 000 g/mol ;
- un deuxième bloc comprenant des unités dérivant de l'acrylate d'éthyle ayant une masse moléculaire moyenne en nombre de 20 000 g/mol ;
- un troisième bloc comprenant des unités dérivant du styrène ayant une masse moléculaire moyenne en nombre de 25 000 g/mol.

Ces copolymères tribloc peuvent se présenter sous forme d'émulsion dans l'eau.

Les copolymères à blocs utilisés selon l'invention peuvent être obtenus par toute méthode connue, que ce soit par polymérisation radicalaire, contrôlée ou non, par polymérisation par ouverture de cycle (notamment anionique ou cationique), par polymérisation anionique ou cationique, ou encore par modification chimique d'un polymère.

De manière préférée, on met en oeuvre des méthodes de polymérisation radicalaire dite vivante ou contrôlée, et de manière particulièrement préférées des méthodes de polymérisation radicalaire contrôlée ou vivante mettant en oeuvre un agent de transfert comprenant un groupe de formule -S-CS-, notamment connues sous les dénominations de RAFT ou MADIX.

On peut citer par exemple la polymérisation anionique et la polymérisation radicalaire contrôlée (voir « New Method of Polymer Synthesis », Blackie Academic & Professional, Londres, 1995, volume 2, page 1, ou Trends Polym. Sci.4, page 183 (1996) de C.J Hawker) qui peut être mise en oeuvre suivant différents procédés comme, par exemple, la polymérisation radicalaire par transfert d'atomes (Atom Transfert Radical Polymerisation ou ATRP) (voir JACS, 117, page 5614 (1995), de Matyjasezwski et al.)

A l'aide des procédés décrits ci-dessus, on peut opérer la préparation d'un premier bloc à partir de monomères

ou d'un mélange de monomères d'initiateurs et/ou d'agents favorisant le contrôle de la polymérisation (agents de transferts à groupes -S-CS-, etc...), puis à la croissance d'un deuxième bloc sur le premier bloc pour obtenir un copolymère dibloc avec des monomères différents de ceux utilisés pour la préparation du bloc précédents, et éventuellement avec ajout d'initiateurs et/ou d'agents favorisant le contrôle de la polymérisation, puis à la croissance d'un troisième bloc à partir sur le copolymère dibloc pour obtenir un copolymère tribloc, etc... Ces procédés de préparations de copolymères à blocs sont connus de l'homme du métier. On mentionne que le copolymère peut présenter en bout de chaîne un groupe de transfert ou un résidu d'un groupe de transfert, par exemple un groupe comprenant un groupe -S-CS- (par exemple issu d'un Xanthate ou d'un dithioester) ou un résidu d'un tel groupe.

On mentionne que les copolymères à tribloc obtenus par des procédés mettant en oeuvre trois séquences successives de polymérisation sont souvent décrits comme des copolymères à blocs A-B-C. Dans le cas où la composition et la masse moléculaire du troisième bloc C sont sensiblement identiques à la composition et la masse moléculaire du premier bloc A (les quantités et proportions de (co)monomères étant sensiblement identiques), les copolymères tribloc peuvent être décrits comme des copolymères tribloc A-B-A' ou par extension ou simplification comme des copolymères tribloc A-B-A.

Ainsi on peut par exemple préparer un copolymère tribloc A-B-A selon l'invention, par un procédé comprenant les étapes suivantes:

- étape a): préparation du bloc A par polymérisation, de préférence radicalaire contrôlée, d'une composition comprenant:

- le styrène,
- une source de radicaux libres, et
- au moins un agent de contrôle, de préférence un agent comprenant un groupe -S-CS-, par exemple un xanthate ou un dithioester,
- étape b) obtention du copolymère dibloc A-B, par croissance du bloc B sur le bloc A, par polymérisation, de préférence radicalaire contrôlée, d'une composition comprenant:

- l'acrylate d'éthyle ou le méthacrylate d'éthyle, et
- éventuellement une source de radicaux libres,
- étape c) obtention du copolymère tribloc A-B-A, par croissance du bloc A sur le dibloc A-B, par polymérisation, de préférence radicalaire contrôlée, d'une composition comprenant:

- l'acrylate d'éthyle ou le méthacrylate d'éthyle, et
- éventuellement une source de radicaux libres,
- étape d) éventuellement destruction ou désactivation d'un groupe de l'agent de contrôle.

Selon d'autres modes de réalisations, on prépare des copolymères tribloc A-B-A en deux séquences de polymérisation, à l'aide d'agents comprenant deux groupes de transfert ou d'agents comprenant un groupe de transfert permettant un transfert à chaque extrémité du groupe, par exemple un trithiocarbonate comprenant un groupe de formule -S-CS-S-. Dans de tels procédés, les blocs A sont parfaitement identiques, et le bloc B comprend généralement un groupe central différent des unités de répétition du bloc B. La mention de la présence d'un groupe central au sein du bloc B, noté par exemple -X-, -X-Z'-X- ou R' plus bas, est souvent omise par soucis de simplification.

Ainsi on peut préparer des copolymères tribloc A-B-A par le procédé suivant:

- étape a'): préparation d'un polymère de formule R-A-X-A-R par polymérisation d'une composition comprenant:

- le styrène
- une source de radicaux libres, et
- au moins un agent de contrôle de formule R-X-R où R, identique ou différent est un groupe organique, et X est un groupe de transfert, l'agent étant de préférence un trithiocarbonate comprenant un groupe -X- de formule -S-CS-S- et de préférence deux groupes R identiques tels que les groupes benzyles (l'agent étant donc par exemple le dibenzyltrithiocarbonate),
- étape b') obtention du copolymère tribloc R-A-B-X-B-A-R (ou plus simplement A-B-A), par croissance du bloc B sur les blocs A par polymérisation, de préférence radicalaire contrôlée, d'une composition comprenant:

- l'acrylate d'éthyle ou le méthacrylate d'éthyle, et
- éventuellement une source de radicaux libres.

On peut également préparer des copolymères tribloc A-B-A par le procédé suivant:

- étape a") : préparation d'un polymère de formule R-A-X-Z'-X-A-R par polymérisation d'une composition comprenant:

- le styrène
- une source de radicaux libres, et
- au moins un agent de contrôle de formule R-X-Z'-X-R où R, identique ou différent, est un groupe organique, Z' est un groupe organique divalent, et X est un groupe de transfert, de préférence un groupe de transfert comprenant un groupe -S-CS-, ledit agent étant par exemple
- un agent comprenant deux groupes xanthates, où -X- est un groupe de formule -S-CS-formant avec -Z'- un groupe de formule -S-CS-O-(xanthate), Z' étant par exemple un groupe de formule - O-$CH_2$-$CH_2$-O-, et R est par exemple un groupe benzyle ou un groupe de formule $H_3C$-OOC-CH($CH_3$) - , ou
- un agent comprenant deux groupes dithioesters, où -X- est un groupe de formule -S-CS-formant avec -Z'- un groupe de formule -S-CS-C-(dithioester), Z' étant par exemple un groupe phényle

ou benzyle, ou un groupe de formule -$CH_2$-$C_6H_5$-$CH_2$-, et R est par exemple un groupe benzyle,

- étape b") obtention du copolymère tribloc R-A-B-X-Z'-X-B-A-R (ou plus simplement A-B-A), par croissance du bloc B sur les blocs A par polymérisation, de préférence radicalaire contrôlée, d'une composition comprenant:

- l'acrylate d'éthyle ou le méthacrylate d'éthyle, et
- éventuellement une source de radicaux libres.

On peut également préparer des copolymères tribloc A-B-A par le procédé suivant:

- étape a'''): préparation d'un polymère de formule Z-X-B-R'-B-X-Z par polymérisation d'une composition comprenant:

- l'acrylate d'éthyle ou le méthacrylate d'éthyle, et
- une source de radicaux libres, et
- au moins un agent de contrôle de formule Z-X-R'-X-Z où Z, identique ou différent, est un groupe organique, R' est un groupe organique divalent, et X est un groupe de transfert, de préférence un groupe de transfert comprenant un groupe -S-CS-, ledit agent étant par exemple:

- un agent comprenant deux groupes xanthates, où -X- est un groupe de formule. -S-CS-formant avec Z- un groupe de formule -O-CS-S-(xanthate), Z- étant par exemple un groupe éthoxy, et - R'- est par exemple un groupe phényle ou benzyle, ou un groupe de formule -$CH_2$-$C_6H_5$-$CH_2$-,
- un agent comprenant deux groupes dithioesters, où -X- est un groupe de formule -S-CS-formant avec Z- un groupe de formule C-CS-S-(dithioester), Z- étant par exemple un groupe phényle

ou benzyle, et -R'- est par exemple un groupe phényle
ou benzyle, ou un groupe de formule -$CH_2$-$C_6H_5$-$CH_2$-,

- étape b''') obtention du copolymère tribloc Z-X-A-B-R'-B-A-X-Z (ou plus simplement A-B-A), par croissance du bloc A sur les blocs B par polymérisation, de préférence radicalaire contrôlée, d'une composition comprenant:

- le styrène, et
- éventuellement une source de radicaux libres,
- étape d''') éventuellement destruction ou désactivation d'un groupe de l'agent de contrôle.

Les polymérisations peuvent être effectuées sous toute forme physique appropriée, par exemple en solution dans un solvant, en émulsion dans l'eau (procédé dit "latex"), en masse, le cas échéant en contrôlant la température et/ou le pH afin de rendre des espèces liquides et/ou solubles ou insolubles.

Les copolymères utilisés selon l'invention, qu'ils soient aléatoires, statistiques ou à blocs, sont avantageusement des copolymères non élastomères.

Par « copolymère non élastomère », on entend généralement un copolymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par « copolymère non élastomère », on désigne un copolymère ayant une recou-

vrance instantanée $R_i$<50% et une recouvrance retardée $R_{2h}$<70% après avoir subi un allongement de 30%. De préférence, $R_i$ est <30% et $R_{2h}$ est <50%. Plus précisément, le caractère non élastomérique du copolymère est déterminé selon le protocole suivant :

On prépare un film de copolymère par coulage d'une solution du copolymère dans une matrice téflonnée puis on le sèche pendant 7 jours dans une ambiance contrôlée à 23±5°C et 50±10% d'humidité relative.
On obtient alors un film d'environ 100 µm d'épaisseur dans lequel dont découpées des éprouvettes rectangulaires (par exemple, à l'emporte-pièce) d'une largeur de 15 mm et d'une longueur de 80 mm.
On impose à ces échantillons sous forme d'éprouvettes une sollicitation de traction à l'aide d'un appareil commercialisé sous la référence Zwick, dans les mêmes conditions de température et d'humidité que pour le séchage.
Les éprouvettes sont étirées à une vitesse de 50 mm/min et la distance entre les mors est de 50 mm, ce qui correspond à la longueur initiale ($1_0$) de l'éprouvette.

On détermine la recouvrance instantanée $R_i$ de la manière suivante :

- on étire l'éprouvette de 30% ($\varepsilon_{max}$), c'est-à-dire environ 0,3 fois sa longueur initiale ($1_0$) ;
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée $R_i$ (en %) est déterminée par la formule suivante :

$$R_i = ((\varepsilon_{max} - \varepsilon_i) / \varepsilon_{max}) * 100$$

Pour déterminer la recouvrance retardée, on mesure l'allongement résiduel de l'éprouvette en pourcentage ($\varepsilon_{2h}$), deux heures après retour à la contrainte nulle.

La recouvrance retardée $R_{2h}$ (en %) est donnée par la formule ci-après :

$$R_{2h} = ((\varepsilon_{max} - \varepsilon_{2h}) / \varepsilon_{max}) * 100$$

A titre indicatif, un copolymère selon un mode de réalisation de l'invention présente une recouvrance instantanée $R_i$ de 10% et une recouvrance retardée $R_{2h}$ de 30%.

La Demanderesse a, en outre, démontré que, pour qu'ils soient suffisamment rigides pour tendre la peau, les copolymères utilisés dans le cadre de l'invention, qu'ils soient aléatoires, statistiques ou à blocs, doivent présenter un rapport en poids entre les unités dérivant du styrène et les unités dérivant du (méth)acrylate d'éthyle supérieur à 1, de préférence supérieur ou égal à 1,5, de préférence supérieur à 2 (notamment supérieur ou égal à 2,01), de préférence supérieur ou égal à 5 . Le rapport peut être également supérieur à 20.

La masse moléculaire moyenne en nombre du copolymère global est généralement supérieure à 10000 g/mol, de préférence supérieure à 50000 g/mol. Cette masse moléculaire ne dépasse pas, de préférence, 600 000 g/mol.

La masse moléculaire moyenne en masse du copolymère global est généralement supérieure à 20000 g/mol. Elle peut être également supérieure à 100000 g/mol et de préférence inférieure à 1 000 000 g/mol.

Selon l'invention, les copolymères décrits ci-dessus sont utilisés dans une composition cosmétique antirides.

Les copolymères utilisés dans le cadre de l'invention sont avantageusement compris, dans les compositions antirides les contenant, en une quantité en matière active allant de 0,1 à 20% en poids, de préférence de 0,5 à 10% en poids.

On entend par matière active le copolymère sans solvant et dénué du milieu de suspension résultant du procédé de polymérisation.

Il est entendu que ladite composition cosmétique comprendra outre le ou les copolymère(s) susmentionné(s), un milieu physiologiquement acceptable, généralement adapté à une application topique sur la peau du visage, c'est-à-dire un milieu compatible avec la peau et éventuellement les cils et les sourcils.

Ledit milieu physiologiquement acceptable est généralement cosmétiquement acceptable, c'est-à-dire qu'il présente une odeur, une couleur et un toucher agréables, compatibles avec une utilisation cosmétique, et ne génère pas d'inconforts inacceptables (picotements, tiraillements, rougeurs) susceptibles de détourner l'utilisateur.

Ledit milieu physiologiquement acceptable comprend généralement une phase aqueuse.

Les compositions dans lesquelles sont incorporées les copolymères spécifiques de l'invention peuvent se

présenter sous toutes les formes galéniques normalement utilisées pour une application topique, notamment sous forme de sérums (c'est-à-dire de solutions aqueuses épaissies) ou sous forme d'émulsions notamment émulsion huile-dans-eau (H/E) ou eau dans huile (E/H) ou multiple (E/H/E ou polyol/H/E ou H/E/H).

Dans le cas où la composition forme une émulsion, elle comprendra une phase grasse.

La phase grasse de cette composition peut être notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique.

Comme corps gras liquides à température ambiante, appelés souvent huiles, utilisables selon l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raison, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale
ou synthétique tels que l'isododécane, les huiles de paraffine et leurs dérivés, la vaseline, les polydécènes, un polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryl ; les esters hydroxylés comme l'isostéaryl lactate, l'octylhydroxystéarate, l'hydroxystéarate d'octyldodécyle, le diiostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polydiméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90%, et mieux de 0,1 à 85% en poids, par rapport au poids total de la composition.

La composition selon l'invention peut également contenir des ingrédients couramment utilisés en cosmétique, tels que des agents épaississants ; des charges, par exemples minérales éventuellement enrobées (telles que l'oxyde de zinc, la silice, l'alumine, le nitrure de bore, le talc, la séricite, le mica) ; des pigments et colorants ; des agents séquestrants ; des parfums ; des agents alcalinisants ou acidifiants ; des conservateurs ; des tensioactifs, et leurs mélanges.

La composition peut contenir également des adjuvants tels que des argiles, l'amidon et ses dérivés, les dispersions aqueuses de copolymères styrène-acide acrylique, les particules de résine de mélamine-formaldéhyde ou d'urée-formaldéhyde, les dispersions aqueuses de polytétrafluoroéthylène, les copolymères vinylpyrrolidone/1-triacontène, les polymères hydrodispersibles contenant des unités à LCST, les cires et les résines de silicone, les microsphères de terpolymère expansé de chlorure de vinylidène, d'acrylonitrile et de méthacrylate commercialisées par la société Expancel, les particules de Nylon, les microbilles de cellulose et les fibres, notamment de Nylon.

Elle peut également contenir des actifs anti-âge à effet complémentaire des copolymères définis précédemment, tels qu'au moins un composé choisi parmi les agents kératolytiques ou prodesquamants, par exemple les α-hydroxyacides, les β-hydroxyacides, les α-cétoacides, les β-cétoacides, les rétinoïdes et leurs esters, en particulier le palmitate de rétinyle et ses dérivés tels que l'acide salicylique et ses dérivés tels que l'acide n-octanoyl-5-salicylique ; les agents hydratants tels que les polyols ; les agents stimulant la synthèse du collagène et/ou de l'élastine ou prévenant leur dégradation ; les agents dépigmentants ou blanchissants comme l'acide kojique, les dérivés de para-aminophénols, l'arbutine et leurs dérivés ; les agents anti-glycation ; les agents stimulant la synthèse de glycoaminoglycannes ; les agents dermo-décontractants ou myorelaxants, tels que l'adénosine et les sels de magnésium et de manganèse ; les agents anti-oxydants et anti-radicalaires ; les vitamines, comme les vitamines C, B3 ou PP, B5, E et les dérivés de ces vitamines et notamment leurs esters ; la vitamine K et ses dérivés (K1, K2) ; les céramides ; la DHEA et ses dérivés et ses précurseurs chimiques tels que la diosgénine et les extraits végétaux en contenant (notamment les extraits végétaux de dioscorées); le coenzyme Q10 ; et leurs mélanges.

Bien entendu, l'homme du métier veillera à choisir ce ou ces éventuels composés complémentaires et/ou leur quantité, de manière telle que les propriétés avantageuses de la composition correspondante selon l'invention ne soient pas ou substantiellement pas altérées par l'adjonction envisagée.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Un autre objet de la présente invention est un procédé cosmétique pour atténuer les rides d'une peau ridée,

tel que le contour de l'oeil, comprenant une étape consistant à appliquer sur ladite peau une composition comprenant, dans un milieu physiologiquement acceptable, au moins un copolymère tel que défini précédemment.

L'application se fait selon les techniques habituelles, par exemple par application de crèmes, de gels, de sérums, de lotions, sur la peau destinée à être traitée, éventuellement suivie d'une étape de rinçage. Dans le cadre de ce procédé, la composition peut être, par exemple, une composition de soin ou de nettoyage, ou une composition de maquillage, en particulier de fond de teint. Il s'agit, de préférence, d'une composition non rincée.

La composition est de préférence appliquée sur le visage et/ou le cou, en particulier sur les zones ridées du visage, et notamment autour des yeux.

D'autres caractéristiques et avantages apparaîtront mieux à la lecture des exemples qui suivent donnés à titre illustratif et non limitatif.

## EXPOSÉ DÉTAILLÉ DE MODES DE RÉALISATION PARTICULIERS

Les exemples suivants proposent un exemple de préparation d'un copolymère utilisable en tant qu'agent tenseur conformément à l'invention ainsi que différentes formulations comprenant ce copolymère.

Exemple 1

Cet exemple illustre la préparation d'un copolymère tribloc comprenant :

- un premier bloc comprenant des unités dérivant du styrène ayant une masse moléculaire moyenne en nombre de 30 000 g/mol ;
- un deuxième bloc constitué d'unités dérivant de l'acrylate d'éthyle ayant une masse moléculaire moyenne en nombre de 10 000 g/mol ;
- un troisième bloc comprenant des unités dérivant du styrène ayant une masse moléculaire moyenne en nombre de 30 000 g/mol.

Le mode opératoire est basé sur un procédé qui peut se décomposer en trois phases distinctes, une première étape qui est l'obtention d'un bloc polystyrène, une deuxième étape qui est la synthèse d'un bloc poly(acrylate d'éthyle) à la suite du premier bloc et une troisième étape qui la synthèse d'un bloc polystyrène à la suite du deuxième bloc, pour obtenir le tribloc polystyrène-b-poly(acrylate d'éthyle)-b-polystyrène.

La synthèse de ce copolymère est réalisée dans un réacteur verré de 2 litres de type SVL. La charge utile maximum de ce type de réacteur est de 1,5 litres. La température interne du réacteur est régulée par un cryostat de type Huber. La mesure de la température est assurée par une sonde pt 100 plongeant dans le réacteur et servant à la régulation. Le mobile d'agitation est une ancre en inox. La vitesse de rotation du mobile est de l'ordre de 200 tr/min. Le réacteur est également équipé d'un dispositif de reflux (réfrigèrent à serpentin) suffisamment efficace pour permettre le reflux des monomères sans pertes de produit.

Le procédé mis en oeuvre est un procédé de polymérisation en émulsion dans l'eau, de type latex.

Etape 1: Préparation du premier bloc.

Cette première étape consiste en la préparation du premier bloc, qui consiste en un copolymère statistique de styrène et d'acide méthacrylique, avec rapport massique (styrène/acide méthacrylique)= 98/2 de masse théorique visée :

$$M_n = 30000 \text{ g/mol.}$$

En pied de cuve, on introduit, à température ambiante, 568,0 g d'eau, 12,0 g de dodécyl sulfate de sodium et 0,95 g de carbonate de sodium $Na_2CO_3$. Le mélange obtenu est agité pendant 30 minutes (200 tr/min) sous azote. La température est ensuite élevée à 75°C, puis on ajoute un mélange 1 comprenant :

- 25,71 g de styrène (St),
- 0,510 g d'acide méthacrylique (AMA), et
- 1,790 g de xanthate $(CH_3)$ $(CO_2CH_3)$ CH-S (C=S) $OCH_2CH_3$.

Le mélange est porté à 85°C, puis on introduit une solution de 0,390 g de persulfate d'ammonium $(NH_4)_2S_2O_8$ solubilisé dans 10,0 g d'eau.

Après 5 minutes, on démarre l'addition d'un mélange 2 comprenant :

- 231,4 g de styrène (St) et
- 4,60 g d'acide méthacrylique (AMA).

On poursuit l'addition pendant 115 minutes. Après addition complète des divers ingrédients, l'émulsion de copolymère obtenue est maintenue à 85°C pendant deux heures.

Un échantillon (~5g) est alors prélevé et analysé par chromatographie d'exclusion stérique (SEC) dans du THF. Sa masse moléculaire moyenne en nombre mesurée Mn est égale à 26600 g/mol en équivalents polystyrène (calibration par des étalons de polystyrène linéaires). Son indice de polymolécularité Mw/Mn est égal à 2,0.

Une analyse de l'échantillon par chromatographie gazeuse révèle que la conversion des monomères est supérieure à 99%.

Etape 2: Préparation du deuxième bloc.

Cette deuxième étape réside dans la synthèse d'un polymère d'acrylate d'éthyle.

On part du copolymère en émulsion obtenu précédemment à l'étape 1 après en avoir prélevé ~5g pour analyse et sans arrêter le chauffage.

On introduit en continu pendant une heure 0,390 g de persulfate d'ammonium $(NH_4)_2S_2O_8$ dilué dans 50,0 g d'eau.

Simultanément pendant une heure, on additionne à 85°C :

- 85,7 g d'acrylate d'éthyle (EA),

Le système est maintenu à cette température pendant deux heures supplémentaires.

Un échantillon (~5g) est alors prélevé et analysé par chromatographie d'exclusion stérique (SEC) dans du THF. Sa masse moléculaire moyenne en nombre mesurée Mn est égale à 37000 g/mol en équivalents polystyrène (calibration par des étalons de polystyrène linéaires). Son indice de polymolécularité Mw/Mn est égal à 1.9.

Une analyse de l'échantillon par chromatographie gazeuse révèle que la conversion des monomères est supérieure à 99%.

Etape 3: Préparation du troisième bloc

Cette première étape consiste en la préparation du troisième bloc, qui consiste en un copolymère statistique de styrène et d'acide méthacrylique, avec rapport massique (styrène/acide méthacrylique)= 98/2 de masse théorique visée : $M_n$ = 30000 g/mol.

On part du copolymère en émulsion obtenu précédemment à l'étape 2 après en avoir prélevé ~5 g pour analyse et sans arrêter le chauffage.

On introduit en continu pendant trois heures 0,390 g de persulfate d'ammonium $(NH_4)_2S_2O_8$ dilué dans 50,0 g d'eau. Ensuite pendant trois heures, on additionne à 85°C un mélange 3 comprenant:

- 50,0 g d'eau,
- 0,95 g de carbonate de sodium $Na_2CO_3$.

Simultanément on ajoute un mélange 4 comprenant :

- 257,1 g de styrène (St), et
- 5,14 g d'acide méthacrylique (AMA)

Après addition complète des divers ingrédients, l'émulsion de copolymère obtenue est maintenue à 85°C pendant une heure.

Puis on introduit 1,20 g de tert-butylbenzylperoxide en une seule fois et on démarre l'addition d'un mélange 5 comprenant :

- 0,600 g d'acide érythorbique
- 20,0 g d'eau.

On poursuit l'addition pendant 60 minutes. Après addition complète des divers ingrédients, l'émulsion est

refroidie à ~25°C pendant une heure.

Un échantillon (~5g) est alors prélevé et analysé par chromatographie d'exclusion stérique (SEC) dans du THF. Sa masse moléculaire moyenne en nombre mesurée Mn est égale à 56800 g/mol en équivalents polystyrène (calibration par des étalons de polystyrène linéaires). Son indice de polymolécularité Mw/Mn est égal à 1.9.

Une analyse de l'échantillon par chromatographie gazeuse révèle que la conversion des monomères est supérieure à 99,8%.

Le produit obtenu est une dispersion dans l'eau du copolymère (latex), d'extrait sec d'environ 44%.

**Exemple 2**

Préparation d'un copolymère tribloc polystyrène-bloc-poly(acrylate d'éthyle)-bloc-polystyrène "$pS_{25k}$-$PEA_{20k}$-$PS_{25k}$"

Le mode opératoire est basé sur un procédé qui peut se décomposer en trois phases distinctes, une première étape qui est l'obtention d'un bloc polystyrène, une deuxième étape qui est la synthèse d'un bloc poly(acrylate d'éthyle) à la suite du premier bloc et une troisième étape qui la synthèse d'un bloc polystyrène à la suite du deuxième bloc, pour obtenir le tribloc polystyrène-b-poly(acrylate d'ethyle)-b-polystyrène.

La synthèse de ce copolymère est réalisée dans un réacteur verré de 2 litres de type SVL. La charge utile maximum de ce type de réacteur est de 1,5 litres. La température interne du réacteur est régulée par un cryostat de type Huber. La mesure de la température est assurée par une sonde pt 100 plongeant dans le réacteur et servant à la régulation. Le mobile d'agitation est une ancre en inox. La vitesse de rotation du mobile est de l'ordre de 200 tr/min. Le réacteur est également équipé d'un dispositif de reflux (réfrigèrent à serpentin) suffisamment efficace pour permettre le reflux des monomères sans pertes de produit.

Le procédé mis en oeuvre est un procédé de polymérisation en émulsion dans l'eau, de type latex.

Etape 1: Préparation d'un premier bloc de polystyrène de masse moléculaire théorique d'environ 25000 g/mol "$pS_{25k}$"

En réalité c'est une synthèse d'un copolymère statistique de styrène et d'acide méthacrylique, avec rapport massique St/AMA = 98/2. Masse théorique visée : $M_n$ = 25000 g/mol. On se réfère à un bloc de polystyrène par soucis de simplicité de la terminologie.

En pied de cuve, on introduit, à température ambiante, 518,2 g d'eau, 6,250 g de dodecyl sulfate de sodium et 0,714 g de carbonate de sodium $Na_2CO_3$. Le mélange obtenu est agité pendant 30 minutes (200 tr/min) sous azote. La température est ensuite élevée à 75°C, puis on ajoute un mélange 1 comprenant :

- 17,86 g de styrène (St),
- 0,357 g d'acide méthacrylique (AMA), et
- 1,486 g de xanthate $(CH_3)$ $(CO_2CH_3)$ CH-S (C=S) $OCH_2CH_3$.

Le mélange est porté à 85°C, puis on introduit une solution de 0,085 g de persulfate de sodium $Na_2S_2O_8$ solubilisé dans 1,70 g d'eau.

Après 5 minutes, on démarre l'addition d'un mélange 2 comprenant :

- 160,7 g de styrène (St) et
- 3,21 g d'acide méthacrylique (AMA).

Simultanément on démarre l'addition d'un mélange 3 comprenant 0,255 g de persulfate de sodium $Na_2S_2O_8$ solubilisé dans 5,10 g d'eau.

On poursuit l'addition pendant 90 minutes. Après addition complète des divers ingrédients, l'émulsion de copolymère obtenue est maintenue à 85°C pendant deux heures.

Un échantillon (~5g) est alors prélevé et analysé par chromatographie d'exclusion stérique (SEC) dans du THF. Sa masse moléculaire moyenne en nombre mésurée Mn est égale à 22000 g/mol en équivalents polystyrène (calibration par des étalons de polystyrène linéaires). Son indice de polymolécularité Mw/Mn est égal à 2,2.

Une analyse de l'échantillon par chromatographie gazeuse révèle que la conversion des monomères est supérieure à 99%.

Etape 2: Préparation d'un deuxième bloc de poly(acrylate d'éthyle) de masse moléculaire théorique d'environ 10000 g/mol pour obtenir un copolymère dibloc polystyrène-bloc-poly (acrylate d'éthyle), " $pS_{25k}$-$pEA_{20k}$"

On part du copolymère en émulsion obtenu précédemment à l'étape 1 après en avoir prélevé ~5 g pour analyse

et sans arrêter le chauffage.
On introduit en continu pendant 90 minutes 0,170 g de persulfate de sodium $Na_2S_2O_8$ dilué dans 3,4 g d'eau.
Simultanément pendant 90 minutes, on additionne à 85°C :

- 142,9 g d'acrylate d'éthyle (EA),

Le système est maintenu à cette température pendant deux heures supplémentaires.
Un échantillon (~5g) est alors prélevé et analysé par chromatographie d'exclusion stérique (SEC) dans du THF. Sa masse moléculaire moyenne en nombre mesurée Mn est égale à 32000 g/mol en équivalents polystyrène (calibration par des étalons de polystyrène linéaires). Son indice de polymolécularité Mw/Mn est égal à 2,6.
Une analyse de l'échantillon par chromatographie gazeuse révèle que la conversion des monomères est supérieure à 99%.

Etape 3: Préparation d'un troisième bloc de polystyrène de masse moléculaire théorique d'environ 25000 g/mol pour obtenir un copolymère tribloc polystyrène-bloc-poly (acrylate d'éthyle) -bloc-polystyrène "$pS_{25k}$-$pEA_{20k}$-$pS_{25k}$"

En réalité c'est une synthèse d'un copolymère statistique de styrène et d'acide méthacrylique. Rapport massique St/AMA = 98/2. Masse théorique visée : $M_n$ = 25000 g/mol. On se réfère à un bloc de polystyrène par soucis de simplicité de la terminologie.
On part du copolymère en émulsion obtenu précédemment à l'étape 2 après en avoir prélevé ~5 g pour analyse et sans arrêter le chauffage.
On introduit en continu pendant deux heures 0,340 g de persulfate de sodium $Na_2S_2O_8$ dilué dans 6,8 g d'eau. Simultanément pendant deux heures, on additionne à 85°C un mélange 4 comprenant:

- 97,90 g d'eau,
- 1,146 g de carbonate de sodium $Na_2CO_3$
- 3,75 g de dodecyl sulfate de sodium.

Simultanément on ajoute un mélange 5 comprenant :

- 178,6 g de styrène (St), et
- 3,57 g d'acide méthacrylique (AMA)

Après addition complète des divers ingrédients, l'émulsion de copolymère obtenue est maintenue à 85°C pendant deux heures.
Puis on introduit 0,500 g de tert-butylbenzylperoxide en une seule fois et on démarre l'addition d'un mélange 6 comprenant :

- 0,250 g d'acide érythorbique
- 5,0 g d'eau.

On poursuit l'addition pendant 60 minutes. Après addition complète des divers ingrédients, l'émulsion est refroidie à ~25°C pendant une heure.
Un échantillon (~5g) est alors prélevé et analysé par chromatographie d'exclusion stérique (SEC) dans du THF. Sa masse moléculaire moyenne en nombre mesurée Mn est égale à 40000 g/mol en équivalents polystyrène (calibration par des étalons de polystyrène linéaires). Son indice de polymolécularité Mw/Mn est égal à 2,9.
Une analyse de l'échantillon par chromatographie gazeuse révèle que la conversion des monomères est supérieure à 99.8%.
Le produit obtenu est une dispersion dans l'eau du copolymère (latex), d'extrait sec d'environ 45%.

Exemple 3 Préparation d'un copolymère tribloc polystyrène-bloc-poly(acrylate d'éthyle)-bloc-polystyrène "$pS_{32,5k}$-$pEA_{5k}$-$pS_{32,5k}$ "

Le mode opératoire est basé sur un procédé qui peut se décomposer en trois phases distinctes, une première étape qui est l'obtention d'un bloc polystyrène, une deuxième étape qui est la synthèse d'un bloc poly(acrylate d'éthyle) à la suite du premier bloc et une troisième étape qui la synthèse d'un bloc polystyrène à la suite du deuxième bloc, pour obtenir le tribloc polystyrène-b-poly(acrylate d'ethyle)-b-polystyrène.
La synthèse de ce copolymère est réalisée dans un réacteur verré de 2 litres de type SVL. La charge utile

maximum de ce type de réacteur est de 1,5 litres. La température interne du réacteur est régulée par un cryostat de type Huber. La mesure de la température est assurée par une sonde pt 100 plongeant dans le réacteur et servant à la régulation. Le mobile d'agitation est une ancre en inox. La vitesse de rotation du mobile est de l'ordre de 200 tr/min. Le réacteur est également équipé d'un dispositif de reflux (réfrigèrent à serpentin) suffisamment efficace pour permettre le reflux des monomères sans pertes de produit.

Le procédé mis en oeuvre est un procédé de polymérisation en émulsion dans l'eau, de type latex.

Etape 1: Préparation d'un premier bloc de polystyrène de masse moléculaire théorique d'environ 32500 g/mol "$pS_{32,5k}$"

En réalité c'est une synthèse d'un copolymère statistique de styrène et d'acide méthacrylique, avec rapport massique St/AMA = 98/2. Masse théorique visée : $M_n$ = 32500 g/mol. On se réfère à un bloc de polystyrène par soucis de simplicité de la terminologie.

En pied de cuve, on introduit, à température ambiante, 515,0 g d'eau, 6,250 g de dodecyl sulfate de sodium et 0,929 g de carbonate de sodium $Na_2CO_3$. Le mélange obtenu est agité pendant 30 minutes (200 tr/min) sous azote. La température est ensuite élevée à 75°C, puis on ajoute un mélange 1 comprenant :

- 23,21 g de styrène (St),
- 0,464 g d'acide méthacrylique (AMA), et
- 1,486 g de xanthate $(CH_3)$ $(CO_2CH_3)$ $CH-S(C=S)OCH_2CH_3$.

Le mélange est porté à 85°C, puis on introduit une solution de 0,085 g de persulfate de sodium $Na_2S_2O_8$ solubilisé dans 1,70 g d'eau.

Après 5 minutes, on démarre l'addition d'un mélange 2 comprenant :

- 208,9 g de styrène (St) et
- 4,18 g d'acide méthacrylique (AMA).

Simultanément on démarre l'addition d'un mélange 3 comprenant 0,255 g de persulfate de sodium $Na_2S_2O_8$ solubilisé dans 5,10 g d'eau.

On poursuit l'addition pendant 80 minutes. Après addition complète des divers ingrédients, l'émulsion de copolymère obtenue est maintenue à 85°C pendant deux heures.

Un échantillon (~5g) est alors prélevé et analysé par chromatographie d'exclusion stérique (SEC) dans du THF. Sa masse moléculaire moyenne en nombre mesurée Mn est égale à 29000 g/mol en équivalents polystyrène (calibration par des étalons de polystyrène linéaires). Son indice de polymolécularité Mw/Mn est égal à 2,2.

Une analyse de l'échantillon par chromatographie gazeuse révèle que la conversion des monomères est supérieure à 99%.

Etape 2: Préparation d'un deuxième bloc de poly(acrylate d'éthyle) de masse moléculaire théorique d'environ 10000 g/mol pour obtenir un copolymère dibloc polystyrène-bloc-poly (acrylate d'éthyle), " $pS_{32,5k}$-$pEA_{5k}$"

On part du copolymère en émulsion obtenu précédemment à l'étape 1 après en avoir prélevé ~5 g pour analyse et sans arrêter le chauffage.

On introduit en continu pendant 90 minutes 0,170 g de persulfate de sodium $Na_2S_2O_8$ dilué dans 3,4 g d'eau.

Simultanément pendant 90 minutes, on additionne à 85°C :

- 35,7 g d'acrylate d'éthyle (EA),

Le système est maintenu à cette température pendant deux heures supplémentaires.

Un échantillon (~5g) est alors prélevé et analysé par chromatographie d'exclusion stérique (SEC) dans du THF. Sa masse moléculaire moyenne en nombre mesurée Mn est égale à 34000 g/mol en équivalents polystyrène (calibration par des étalons de polystyrène linéaires). Son indice de polymolécularité Mw/Mn est égal à 2,3.

Une analyse de l'échantillon par chromatographie gazeuse révèle que la conversion des monomères est supérieure à 99%.

Etape 3: Préparation d'un troisième bloc de polystyrène de masse moléculaire théorique d'environ 32500 g/mol pour obtenir un copolymère tribloc polystyrène-bloc-poly (acrylate d'éthyle)-bloc-polystyrène "$pS_{35,5k}$-$pEA_{5k}$-$pS_{32,5k}$ "

En réalité c'est une synthèse d'un copolymère statistique de styrène et d'acide méthacrylique. Rapport massique

St/AMA = 98/2. Masse théorique visée : $M_n$ = 32500 g/mol. On se réfère à un bloc de polystyrène par soucis de simplicité de la terminologie.

On part du copolymère en émulsion obtenu précédemment à l'étape 2 après en avoir prélevé ~5 g pour analyse et sans arrêter le chauffage.

On introduit en continu pendant deux heures 0,340 g de persulfate de sodium $Na_2S_2O_8$ dilué dans 6,8 g d'eau. Simultanément pendant deux heures, on additionne à 85°C un mélange 4 comprenant:

- 104,4 g d'eau,
- 1,468 g de carbonate de sodium $Na_2CO_3$
- 3,75 g de dodecyl sulfate de sodium.

Simultanément on ajoute un mélange 5 comprenant :

- 1232,1 g de styrène (St), et
- 4,64 g d'acide méthacrylique (AMA)

Après addition complète des divers ingrédients, l'émulsion de copolymère obtenue est maintenue à 85°C pendant deux heures.

Puis on introduit 0,500 g de tert-butylbenzylperoxide en une seule fois et on démarre l'addition d'un mélange 6 comprenant :

- 0,250 g d'acide érythorbique
- 5,0 g d'eau.

On poursuit l'addition pendant 60 minutes. Après addition complète des divers ingrédients, l'émulsion est refroidie à ~25°C pendant une heure.

Un échantillon (~5g) est alors prélevé et analysé par chromatographie d'exclusion stérique (SEC) dans du THF. Sa masse moléculaire moyenne en nombre mesurée Mn est égale à 50000 g/mol en équivalents polystyrène (calibration par des étalons de polystyrène linéaires). Son indice de polymolécularité Mw/Mn est égal à 2,6.

Une analyse de l'échantillon par chromatographie gazeuse révèle que la conversion des monomères est supérieure à 99.8%.

Le produit obtenu est une dispersion dans l'eau du copolymère (latex), d'extrait sec d'environ 45%.

**Exemple 4- Composition cosmétique sous forme de sérum.**

*Phase A

| | |
|---|---|
| Eau | 80,05 g |
| Copolymère méthyl vinyl éther/anhydride maléique (Stabilize QM d'ISP) | 0,20 g |
| Gomme de xanthane | 0,20 g |
| p-hydroxybenzoate de méthyle | 0,20 g |
| Phénoxyéthanol | 0,35 g |

*Phase B

| | |
|---|---|
| Triéthanolamine | 0,20 g |
| Eau | 1 g |

*Phase C

| | |
|---|---|
| Polyacrylamide et isoparaffine $C_{13}$-$C_{19}$ et laureth-7 (Sepigel 305 de Seppic) | 1,00 g |
| Diazolidinylurée | 0,30 g |

*Phase D

Emulsion de copolymère tribloc préparé selon l'exemple 1: 16,50 g à 42,6% de matière active.

Mode opératoire

On chauffe à 75°C environ, sous agitation, la phase A, puis on verse la phase B dans la phase A. Ensuite, on arrête le chauffage tout en maintenant l'agitation jusqu'au retour de la température ambiante et l'on ajoute alors les phases C et D. Une légère agitation est ensuite maintenue pendant 30 minutes.

Cette composition a été testée sur un panel de femmes âgées de 40 à 60 ans présentant des rides et ridules au niveau du contour de l'oeil. Après application de cette composition, il a été observé un effet de lissage mécanique des rides et des ridules. De plus, cette composition a pour effet de diminuer la visibilité du microrelief de la peau donnant l'effet d'un voile « soft focus » qui affine le grain de la peau. L'on ne constate pas avec cette composition d'apparition d'un film laqué inesthétique.

**EXEMPLE 4- Composition cosmétique (essence)**

*Phase A

| | |
|---|---|
| Cyclohexasiloxane | 5g |
| Polyisobutène hydrogéné | 5g |
| Diméthicone PEG-7 phosphate | 2g |

*Phase B

| | |
|---|---|
| Gomme de xanthane | 0,4 g |
| Hydroxyde de sodium | 0,3 g |
| p-hydroxybenzoate d'isobutyle | 0,021 g |
| Eau | 69,15 g |
| Carboxypolyméthylène | 0,4 g |
| Copolymère acrylique (Pemulen TR1 de Noveon) | 0,25 g |
| p-hydroxybenzoate de propyle | 0,021 g |
| p-hydroxybenzoate de butyle | 0,042 g |
| p-hydroxybenzoate de méthyle | 0,174 g |
| p-hydroxybenzoate d'éthyle | 0,042 g |
| Phénoxyéthanol | 0,7 g |

*Phase C.

Emulsion de copolymère tribloc préparé selon l'exemple 1: 16,50 g à 42,6% de matière active.

*Mode opératoire.

On chauffe à 75°C environ, sous agitation, la phase B, jusqu'à obtention d'un gel homogène.
On chauffe la phase A à environ 75°C. On réalise une émulsion en incorporant la phase A dans la phase B.
A une température de 40-45°C, on incorpore la phase C dans l'émulsion précédemment obtenue et on maintient l'agitation jusqu'à refroidissement complet.
Cette composition a été testée sur un panel de femmes âgées de 40 à 60 ans présentant des rides et ridules au niveau du contour de l'oeil. Après application de cette composition, il a été observé un effet de lissage mécanique des rides et des ridules. De plus, cette composition a pour effet de diminuer la visibilité du microrelief de la peau donnant l'effet d'un voile « soft focus » qui affine le grain de la peau. L'on ne constate pas avec cette composition d'apparition d'un film laqué inesthétique.

**Exemple 5 comparatif**

A titre comparatif, un copolymère dibloc comprenant :

- un premier bloc constitué d'unités dérivant du styrène ayant une masse moléculaire moyenne en nombre de 5000 g/mol ;
- un deuxième bloc constitué d'unités dérivant de l'acrylate d'éthyle ayant une masse moléculaire moyenne en nombre de 60 000 g/mol,

dans lequel le rapport entre les unités dérivant du styrène et les unités dérivant de l'acrylate d'éthyle est inférieur à 1, a été testé pour déterminer son effet tenseur. Il a été constaté que ce copolymère ne présentait pas d'effet tenseur.

**Exemple 6**

On prépare une composition à effet tenseur de la peau comprenant les ingrédients suivants :

- Mélange de tartrate de dialkyle(C14-C15), d'alcool cetylstéarylique, d'alcool laurylique oxyéthyléné (25 OE) oxypropyléné (25 OP) (Cosmacol PSE de la société SASOL) 1,5 g
- Mélange de mono, distéarate de glycéryle et de stéarate de polyéthylène glycol (100 OE) (ARLACEL 165FL de chez UNIQEMA) 2 g
- Alcool stéarylique 1 g
- Cyclohexasiloxane 10 g
- Emulsion aqueuse de copolymère tribloc de l'exemple 2 15,3 g
- Acide polyacrylamidométhyl propane sulfonique neutralisé partiellement par l'ammoniaque et réticulé (Hostacerin AMPS de chez CLARIANT) 0,4 g
- Gomme de Xanthane 0,2 g
- Sel disodique de l'acide éthylène diamine tétracétique 0,05 g
- Conservateurs qs
- Eau qsp 100 g

**Exemple 6**

On prépare une composition à effet tenseur de la peau comprenant les ingrédients suivants :

Diméthicone copolyol phosphate
(Pecosil PS 100 de chez Phoenix Chemical) 2 g
- Cyclohexasiloxane 5 g
- Isoparaffine hydrogénée
(Huile de Parleam de chez NOF) 5 g
- Polymère carboxyvinylique
(Carbopol 980 de chez NOVEON) 0,4 g
- Soude 0,3 g
- Gomme de Xanthane 0,4 g
- Copolymère acide acrylique / acrylate
d'alkyle(C10-C30) (Pemulen TR2 de chez NOVEON) 0,25 g
- Emulsion aqueuse de copolymère tribloc
de l'exemple 3 15,3 g
- Conservateurs qs
- Eau qsp 100 g

**Revendications**

1. Utilisation cosmétique en tant qu'agent tenseur de la peau dans une composition cosmétique d'un copolymère comprenant des unités dérivant du styrène et des unités dérivant du (méth)acrylate d'éthyle, dans lequel le rapport en poids entre les unités dérivant du styrène et les unités dérivant du (méth)acrylate d'éthyle est supérieur ou égal à 1.

2. Utilisation selon la revendication 1, dans laquelle la composition cosmétique est une composition antirides.

3. Utilisation selon la revendication 1 ou 2, dans laquelle le rapport en poids entre les unités dérivant du styrène et les unités dérivant du (méth)acrylate d'éthyle est supérieur à 2.

4. Utilisation selon la revendication 1 ou 2, dans laquelle le rapport en poids entre les unités dérivant du styrène et les unités dérivant du (méth)acrylate d'éthyle est supérieur à 5.

5. Utilisation selon la revendication 1 ou 2, dans laquelle le rapport en poids entre les unités dérivant du styrène et les unités dérivant du (méth)acrylate d'éthyle est supérieur à 20.

6. Utilisation selon l'une quelconque des revendications 1 à 5, dans laquelle le copolymère est un copolymère aléatoire.

7. Utilisation selon l'une quelconque des revendications 1 à 5, dans laquelle le copolymère est un copolymère à blocs.

8. Utilisation selon la revendication 7, dans laquelle le copolymère à blocs est un copolymère linéaire à blocs du type $A\text{-}[B\text{-}A]_n$ ou $B\text{-}[A\text{-}B]_n$ ou $[A\text{-}B]_n$ dans lequel A est un bloc comprenant au moins 50% en poids d'unités dérivant du styrène, B est un bloc comprenant au moins 50% en poids d'unités dérivant du (méth)acrylate d'éthyle, et n est un nombre supérieur ou égal à 1.

9. Utilisation selon la revendication 8, dans laquelle le copolymère à blocs est un copolymère tribloc.

10. Utilisation selon la revendication 9, dans laquelle le copolymère tribloc comprend :

    - un premier bloc comprenant des unités dérivant du styrène ayant une masse moléculaire moyenne en nombre de 30 000 g/mol ;
    - un deuxième bloc constitué d'unités dérivant de l'acrylate d'éthyle ayant une masse moléculaire moyenne en nombre de 10 000 g/mol ;
    - un troisième bloc comprenant des unités dérivant du styrène ayant une masse moléculaire moyenne en nombre de 30 000 g/mol.

11. Utilisation selon l'une quelconque des revendications 1 à 10, dans laquelle la masse moléculaire moyenne en nombre du copolymère global est supérieure à 10000 g/mol, de préférence supérieure à 50000 g/mol.

12. Utilisation selon l'une quelconque des revendications 1 à 11, dans laquelle la composition est adaptée à une application topique sur la peau du visage.

13. Procédé cosmétique pour atténuer les rides d'une peau ridée, comprenant une étape consistant à appliquer sur ladite peau une composition comprenant, dans un milieu physiologiquement acceptable, au moins un copolymère tel que défini selon l'une quelconque des revendications 1 à 12.

14. Procédé de traitement cosmétique selon la revendication 13, dans lequel l'application se fait sur le contour de l'oeil.

## Claims

1. Cosmetic use, as a skin-tensioning agent in a cosmetic composition, of a copolymer comprising units derived from styrene and units derived from ethyl (meth)acrylate, in which the weight ratio between the units derived from styrene and the units derived from ethyl (meth)acrylate is greater than or equal to 1.

2. Use according to Claim 1, in which the cosmetic composition is an anti-wrinkle composition.

3. Use according to Claim 1 or 2, in which the weight ratio between the units derived from styrene and the units derived from ethyl (meth)acrylate is greater than 2.

4. Use according to Claim 1 or 2, in which the weight ratio between the units derived from styrene and the units derived from ethyl (meth)acrylate is greater than 5.

5. Use according to Claim 1 or 2, in which the weight ratio between the units derived from styrene and the units derived from ethyl (meth)acrylate is greater than 20.

6. Use according to any one of Claims 1 to 5, in which the copolymer is a random copolymer.

7. Use according to any one of Claims 1 to 5, in which the copolymer is a block copolymer.

8. Use according to Claim 7, in which the block copolymer is a linear block copolymer of the type $A\text{-}[B\text{-}A]_n$ or $B\text{-}[A\text{-}B]_n$ or $[A\text{-}B]_n$ in which A is a block comprising at least 50% by weight of units derived from styrene, B is a block comprising at least 50% by weight of units derived from ethyl (meth) acrylate, and n is a number greater than or equal to 1.

9. Use according to Claim 8, in which the block copolymer is a triblock copolymer.

10. Use according to Claim 9, in which the triblock copolymer comprises:

- a first block comprising units derived from styrene with a number-average molecular mass of 30 000 g/mol;
- a second block consisting of units derived from ethyl acrylate with a number-average molecular mass of 10 000 g/mol;
- a third block comprising units derived from styrene with a number-average molecular mass of 30 000 g/mol.

11. Use according to any one of Claims 1 to 10, in which the number-average molecular mass of the overall copolymer is greater than 10 000 g/mol and preferably greater than 50 000 g/mol.

12. Use according to any one of Claims 1 to 11, in which the composition is suitable for topical application to facial skin.

13. Cosmetic process for reducing the wrinkles of wrinkled skin, comprising a step that consists in applying to the said skin a composition comprising, in a physiologically acceptable medium, at least one copolymer as defined according to any one of Claims 1 to 12.

14. Cosmetic treatment process according to Claim 13, in which the application is made to the contour of the eyes.

## Patentansprüche

1. Kosmetische Verwendung eines Copolymers, das von Styrol abgeleitete Einheiten und von Ethyl(meth)acrylat abgeleitete Einheiten enthält, wobei das Gewichtsverhältnis der von Styrol abgeleiteten Einheiten und der von Ethyl (meth)acrylat abgeleiteten Einheiten größer oder gleich 1 ist, als Straffungsmittel für die Haut in einer kosmetischen Zusammensetzung.

**2.** Verwendung nach Anspruch 1, wobei es sich bei der kosmetischen Zusammensetzung um eine Zusammensetzung gegen Falten handelt.

**3.** Verwendung nach Anspruch 1 oder 2, wobei das Gewichtsverhältnis der von Styrol abgeleiteten Einheiten und der von Ethyl(meth)acrylat abgeleiteten Einheiten größer 2 ist.

**4.** Verwendung nach Anspruch 1 oder 2, wobei das Gewichtsverhältnis der von Styrol abgeleiteten Einheiten und der von Ethyl(meth)acrylat abgeleiteten Einheiten größer 5 ist.

**5.** Verwendung nach Anspruch 1 oder 2, wobei das Gewichtsverhältnis der von Styrol abgeleiteten Einheiten und der von Ethyl(meth)acrylat abgeleiteten Einheiten größer 20 ist.

**6.** Verwendung nach einem der Ansprüche 1 bis 5, wobei das Copolymer ein statistisches Copolymer ist.

**7.** Verwendung nach einem der Ansprüche 1. bis 5, wobei das Copolymer ein Blockcopolymer ist.

**8.** Verwendung nach Anspruch 7, wobei das Blockcopolymer ein lineares Copolymer mit Blöcken vom Typ A-$[B-A]_n$ oder B-$[A-B]_n$ oder $[A-B]_n$ ist, wobei A ein Block ist, der mindestens 50 Gew.-% von Styrol abgeleitete Einheiten enthält, B ein Block ist, der mindestens 50 Gew.-% von Ethyl(meth)acrylat abgeleitete Einheiten enthält, und n eine Zahl größer oder gleich 1 ist.

**9.** Verwendung nach Anspruch 8, wobei das Blockcopolymer ein Dreiblock-Copolymer ist.

**10.** Verwendung nach Anspruch 9, wobei das Dreiblock-Copolymer enthält:

- einen ersten Block, der von Styrol abgeleitete Einheiten aufweist und eine zahlenmittlere Molmasse von 30 000 g/mol besitzt;
- einen zweiten Block, der aus von Ethylacrylat abgeleiteten Einheiten gebildet wird und eine zahlenmittlere Molmasse von 10 000 g/mol aufweist;
- einen dritten Block, der von Styrol abgeleitete Einheiten enthält und eine zahlenmittlere Molmasse von 30 000 g/mol besitzt.

**11.** Verwendung nach einem der Ansprüche 1 bis 10, wobei die zahlenmittlere Molmasse des Copolymers insgesamt größer 10 000 g/mol ist und vorzugsweise über 50 000 g/mol liegt.

**12.** Verwendung nach einem der Ansprüche 1 bis 11, wobei die Zusammensetzung für eine topische Anwendung auf die Haut des Gesichts geeignet ist.

**13.** Kosmetisches Verfahren, um Falten faltiger Haut zu mildern, das einen Schritt umfasst, der darin besteht, auf die Haut eine Zusammensetzung aufzutragen, die in einem physiologisch akzeptablen Medium mindestens ein Copolymer enthält, wie es in einem der Ansprüche 1 bis 12 definiert ist.

**14.** Verfahren zur kosmetischen Behandlung nach Anspruch 13, wobei das Auftragen an der Augenkontur erfolgt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9829092 A
- EP 1038519 A
- FR 2828810
- FR 2822676


### Littérature non-brevet citée dans la description


- New Method of Polymer Synthesis. Blackie Academic & Professional, 1995, vol. 2, 1
- **C.J HAWKER.** *Trends Polym. Sci.,* 1996, vol. 4, 183
- **MATYJASEZWSKI.** *JACS,* 1995, vol. 117, 5614